# EUROPÄISCHE PATENTSCHRIFT

(11) **EP 1 454 609 B1**
(45) Veröffentlichungstag und Bekanntmachung des Hinweises auf die Patenterteilung: **24.10.2012**
(21) Anmeldenummer: 04003326.8
(22) Anmeldetag: 14.02.2004
(51) Int. Cl.: A61J 1/20

(54) **Transfervorrichtung**
Transfer device
Dispositif de transfert

(30) Priorität: 05.03.2003 DE 10309796
(43) Veröffentlichungstag der Anmeldung: 08.09.2004
(73) Patentinhaber: CSL Behring GmbH, 35041 Marburg (DE)
(72) Erfinder: Jansen, Hubert, Dr., 52223 Stolberg (DE); Wortmann, Uwe, 35043 Marburg (DE)

(56) Entgegenhaltungen:
- EP-A- 1 145 702
- EP-A1- 0 499 481
- WO-A-99/08036
- US-A- 4 883 483

## Beschreibung

Die Erfindung betrifft eine Transfervorrichtung, insbesondere für ein unter Unterdruck stehendes Behältnis, mit einer Aufnahmekappe, zur Aufnahme eines mittels eines elastischen Stopfens verschlossenen Wulstes des Behältnisses, wobei die Aufnahmekappe einen Randabschnitt zum Zentrieren des Wulstes in dessen in die Aufnahmekappe eingesteckter Stellung und einen Deckelabschnitt aufweist, mit dem Deckelabschnitt ein zentraler Einstechdorn verbunden ist, der in den von der Aufnahmekappe umschlossenen Raum ragt, wobei der Einstechdorn beim Einstecken des Wulstes in die Aufnahmekappe den Stopfen durchsticht, sowie der Einstechdorn einen, diesen längs durchsetzenden Strömungskanal für ein Fluid aufweist, der durch den Deckelabschnitt nach außen geführt ist.

Eine derartige Transfervorrichtung ist beispielsweise aus der US 2002 / 00 87 141 A1 bekannt. Bei dieser weist der Einstechdorn, abgesehen von seinem spitzen Ende, einen konstanten Außendurchmesser auf. Auch aus der US 4 883 483 ist eine solche Transfervorrichtung bekannt.

Insbesondere bei Transfervorrichtungen, bei denen ein Fluid in das unter Unterdruck stehende Behältnis überführt werden soll, beispielsweise um mittels einer medizinischen Flüssigkeit eine medizinische Substanz zu lösen, ist es wichtig, dass der in den elastischen Stopfen eingestochene Einstechdorn zum Stopfen hin abgedichtet ist. Nur so kann der Unterdruck im Behältnis aufrecht erhalten werden. Dies ist erforderlich, um das Fluid in das Behältnis einzusaugen. Bei einem vorzeitigen Druckausgleich im Behältnis gelangt nur eine unzureichende Menge des Fluids in das Behältnis. Das Mischungsverhältnis stimmt damit nicht, womit der Inhalt des Behältnisses zu verwerfen ist.

In aller Regel sind die Behältnisse, die im Zusammenhang mit derartigen Transfervorrichtungen verwendet werden, als Glasfläschchen ausgebildet.

Bei derartigen Transfervorrichtungen gelangt beim Einstecken des Behältnisses in die Transfervorrichtung das spitze Ende des Einstechdorns in Kontakt mit dem Stopfen, bevor der Randabschnitt der Aufnahmekappe den Wulst des Behältnisses kontaktiert und damit den Wulst und infolge dessen den Stopfen relativ zum Einstechdorn zentrieren kann. Beim außermittigen Positionieren des Stopfens relativ zur Spitze des Einstechdorns, führt die Einsteckbewegung und damit die Führung des Wulstes des Behältnisses mittels des Randabschnittes der Aufnahmekappe dazu, dass der außermittig in den Stopfen eingesteckte Einstechdorn nunmehr in seine mittige Position bewegt wird. Die Folge ist ein Riss im Gummi, der von der Aufsetzstelle des Einstechdorns zum Zentrum des Stopfens reicht. Der Einstechdorn, der einen konstanten Durchmesser aufweist, ist nicht geeignet, diesen Riss abzudichten. Infolge dessen entsteht die beschriebene Leckage.

Aufgabe der vorliegenden Erfindung ist es, eine Transfervorrichtung der eingangs genannten Art so weiterzubilden, dass bei durchstochenem Stopfen ein fluiddichter Kontakt von Einstechdorn und Stopfen selbst dann gewährleistet ist, wenn der Einstechdorn außermittig auf den Stopfen aufgesetzt wurde.

Gelöst wird die Aufgabe mit einer Transfervorrichtung mit den Merkmale des 1. Anspruchs.

Es ist somit vorgesehen, dass neben dem Einstechabschnitt, dem die Funktion zukommt, den Stopfen zu durchstechen, ein in Einsteckrichtung hinterer Dichtabschnitt vorgesehen ist, der den beim außermittigen Aufsetzen der Transfervorrichtung auf den Stopfen entstehenden Riss im Stopfen abdichtet. Infolge dessen durchsetzt der Einstechdorn abgedichtet den Stopfen, womit eine Leckage verhindert wird.

Vorteilhaft ist der Durchmesser des Einstechabschnittes relativ gering, wobei es sich hierbei durchaus um Nadelstärke handeln kann.

Vorzugsweise ist die Transfervorrichtung aus Kunststoff hergestellt, insbesondere als Spritzgussteil. Hierbei wird es als besonders zweckmäßig angesehen, wenn die Transfervorrichtung im Wesentlichen rotationssymmetrisch ausgebildet ist. Bei dieser Gestaltung bzw. Art der Herstellung lässt sich der Rand der Aufnahmekappe vorteilhaft mit einem Rücksprung gestalten, der den Wulst in der in die Aufnahmekappe eingesteckten Stellung des Wulstes hintergreift. Um die Zentrierung des Einstechdornes relativ zum Stopfen präzise zu verwirklichen, sollte der axiale Abstand von Rücksprung und Dichtabschnitt kleiner sein als der axiale Abstand von Rücksprung und dem Deckel zugewandter Fläche des Stopfens bei in die Aufnahmekappe eingesteckter Stellung des Wulstes.

Der Einstechdorn kann auf unterschiedliche Art und Weise ausgebildet sein, um die gemäß der Erfindung vorgesehene Abdichtung des Risses im Stopfen zu gewährleisten.

Die Länge des Dichtabschnittes ist derart bemessen, dass der Dichtabschnitt, in der die Aufnahmekappe eingesteckten Stellung des Wulstes, in den Stopfen eindringt. Die Abdichtung des Stopfens erfolgt somit unter einer gewissen Vorspannung, mit der Folge, dass der im Stopfen entstandene Riss, unter Einwirkung der Druckkräfte des Einstechdorns auf den Stopfen, zusammengedrückt wird.

Der Einstechabschnitt sich erweitert zum Dichtabschnitt hin konisch . Die Abdichtung des Risses im Stopfen erfolgt somit aufgrund der Konizität des Dichtabschnittes.

Es ist genauso denkbar, den Einstechdorn stufenförmig auszubilden, wobei sich an die Stufe zwischen Einstechabschnitt und Dichtabschnitt der sich konisch erweiternde Dichtabschnitt anschließt. Die Abdichtung des Risses im Stopfen erfolgt hierbei über die konische Fläche des Dichtabschnitts. Grundsätzlich besteht die Möglichkeit, im Anschluss an den konischen Dichtabschnitt einen weiteren Bereich des Dichtabschnitts stufenförmig anzuschließen. Die Abdichtung des Stopfens erfolgt in diesem Fall einerseits radial über den konischen Bereich des Dichtabschnittes, andererseits axial durch den sich hieran anschließenden abgestuften Bereich des Dichtabschnittes.

Weitere Merkmale der Erfindung sind in der Beschreibung der Figuren und den Figuren selbst dargestellt.

In der Zeichnung der Figuren ist die Erfindung anhand mehrerer Ausführungsbeispiele veranschaulicht, ohne hierauf beschränkt zu sein. Es zeigt:
- Fig. 1: eine räumliche Ansicht eines ersten Ausführungsbeispieles der erfindungsgemäßen Transfervorrichtung, schräg von unten gesehen,
- Fig. 2: einen Längsmittelschnitt durch die in Figur 1 gezeigte Transfervorrichtung,
- Fig. 3: die Transfervorrichtung gemäß der Figuren 1 und 2 zum Zeitpunkt des exzentrischen Aufsetzens auf den Stopfen, der in ein Glasfläschchen eingesteckt ist, in einem Längsmittelschnitt veranschaulicht,
- Fig. 4: eine Schnittdarstellung der Anordnung gemäß Figur 3, bei in den Stopfen eingestecktem, zentriertem Einstechdorn,
- Fig. 5: eine Schnittdarstellung der Anordnung gemäß der Figuren 3 und 4, bei vollständig auf das Glasfläschchen aufgesteckter Transfervorrichtung,
- Fig. 6: eine räumliche Ansicht eines unbeanspruchten Beispieles der Transfervorrichtung, schräg von unten gesehen,
- Fig. 7: die Transfervorrichtung gemäß Figur 6, in einem Längsmittelschnitt,
- Fig. 8: eine räumliche Ansicht eines zweiten unbeanspruchten zweiten Beispieles der Transfervorrichtung, schräg von unten ges5ehen,
- Fig. 9: die in Figur 8 gezeigte Transfervorrichtung in einem Längsmittelschnitt.

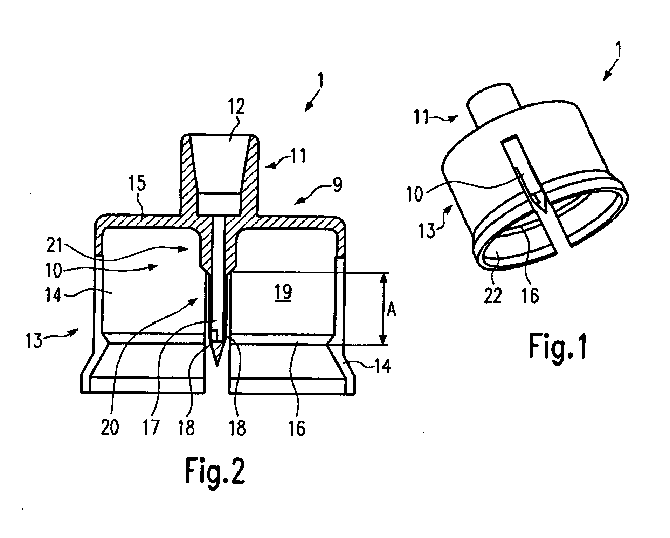

Die Transfervorrichtung 1 gemäß dem in den Figuren 1 bis 5 gezeigten ersten Ausführungsbeispiel ist als Spritzgussteil aus Kunststoff hergestellt. Sie findet insbesondere Verwendung für ein im Inneren unter Unterdruck stehendes B.ehältnis, das als Glasfläschchen 2 ausgebildet ist.

Das Glasfläschchen 2 weist im Anschluss an den Flaschenhals 3 einen Wulst 4 auf, in den ein elastischer Stopfen 5 eingesteckt ist. Der Außendurchmesser des Stopfens 5 entspricht dem des Wulstes 4. Sicher gehalten ist der Stopfen 5 im Wulst 4 mittels einer dünnwandigen Kappe 6, die den Stopfen 5 und den Wulst 4 umschließt, bis auf eine zentrale Öffnung 7 in der Kappe 6 im Bereich der Symmetrieachse von Kappe 6 und Glasfläschchen 2. Der in das Glasfläschchen 2 eingesteckte Bereich des Stopfens 5 ist ringförmig ausgebildet, so dass, dem Inneren des Glasfläschchens 2 zugewandt, eine Ausnehmung 8 im Stopfen 5 gebildet ist. In diesem Bereich weist der Stopfen 5 eine Stärke auf, die derjenigen des Stopfens im Bereich des Außenrandes entspricht.

Die Transfervorrichtung 1 ist durch eine Aufnahmekappe 9, einen Einstechdorn 10 und einen Anschlussstutzen 11 gebildet. Der Anschlussstutzen 11 ist mit einer sich konisch verjüngenden Ausnehmung 12 versehen, die der Aufnahme beispielsweise des Spritzenkonus einer Einmalspritze dient. Andererseits ist der Anschlussstutzen 11 geeignet, eine andere, komplementäre Transfervorrichtung abgedichtet aufzunehmen, beispielsweise wie es in der US 2002/0087,141 A1 bezüglich grundsätzlichem Aufbau und Verwendung der Transfervorrichtung beschrieben ist. In diesem Fall dient die Gesamtvorrichtung dem Überleiten einer medizinischen Flüssigkeit, die sich in einem ersten Glasfläschchen befindet, in ein zweites Glasfläschchen, vorliegend das Glasfläschchen 2, das unter Unterdruck steht und in dem sich beispielsweise eine zu lösende, medizinische Substanz befindet. Nach dem Lösen dieser Substanz wird die andere Transfervorrichtung von der Transfervorrichtung 1 getrennt und es kann, mittels der in den Anschlussstutzen 11 eingesteckten Einmalspritze, nachdem das Fläschchen 2 auf den Kopf gestellt ist, die gelöste Substanz diesem Fläschchen entnommen werden.

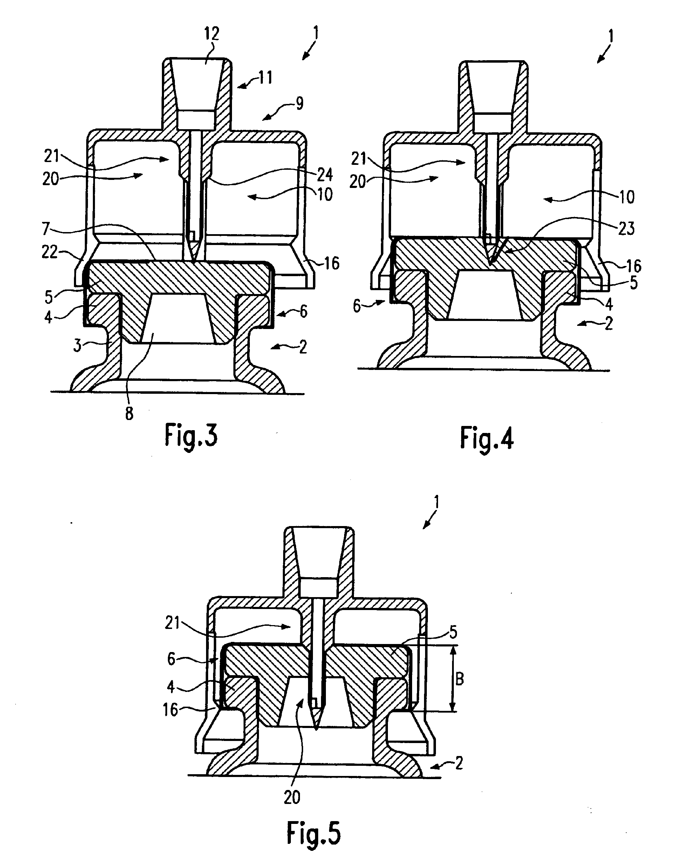

Die Aufnahmekappe 9 der Transfervorrichtung 1 besitzt einen Randabschnitt 13 zum Zentrieren des Wulstes 4 in dessen in die Aufnahmekappe 9 eingesteckten Stellung. Axial ist der Randabschnitt 13 mit vier, sich jeweils über einen Sektor von 90° erstreckenden Lappen 14 versehen, die radial nachgiebig bezüglich eines sich an die Randabschnitte 13 anschließenden Deckelabschnittes 15 der Aufnahmekappe 9 gelagert sind, so dass sie beim Einstecken des Glasfläschchen 2 in die Transfervorrichtung 1 nach außen federn können. Der Randabschnitt 13 ist mit einem nach innen gerichteten Rücksprung 16 versehen. Dieser hintergreift den Wulst 4 des Fläschchens 2 in dessen in die Aufnahmekappe 9 eingesteckten Stellung (wie es in Figur 5 veranschaulicht ist). Dieser Rücksprung 16 ist parallel zum plattenförmigen Deckelabschnitt 15 angeordnet.

Bei der rotationssymmetrisch ausgebildeten Transfervorrichtung 1 ist mit dem Deckelabschnitt 15 der Einstechdorn 10 verbunden. Dessen dem Deckelabschnitt 15 abgewandtes Ende ist spitz ausgebildet. Den Einstechdorn 10 durchsetzt ein Strömungskanal 17, der im Bereich der Spitze des Einstechdorns 10 mit radialen Öffnungen 18 versehen ist und mit seinem anderen Ende mit der Ausnehmung 12 in Verbindung steht. Der Einstechdorn 10 ragt damit in den von der Aufnahmekappe 9 umschlossenen Raum 19.

Der Einstechdorn 10 weist, bezogen auf dessen Einstechrichtung, einen vorderen Einstechabschnitt 20 und einen hinteren, im Durchmesser größeren Dichtabschnitt 21 auf. Der Übergang vom Einstechabschnitt 20 zum Dichtabschnitt 21 ist stufenförmig. Die Länge des Dichtabschnittes 21 ist derart bemessen, dass dieser, in der in die Aufnahmekappe 9 eingesteckten Stellung des Wulstes 4, in den Stopfen 5 eindringt. Bedingt ist dies aufgrund des axialen Abstandes A von Rücksprung 16 und Dichtabschnitt 21, der kleiner ist als der axiale Abstand B von Rücksprung 16 und dem Deckelabschnitt 15 zugewandter Fläche des Stopfens 5, bei in die Aufnahmekappe 9 eingesetzter Stellung des Wulstes 4.

Die vorbezeichneten Details sind der Darstellung der Figuren 3 bis 5 zu entnehmen, die den Vorgang beim Einstecken des Glasfläschchens 2 in die Transfervorrichtung 1 veranschaulichen: Figur 3 zeigt die mit der Spitze des Einstechdorns 10 exzentrisch auf den Stopfen 5 aufgesetzte Transfervorrichtung 1. Zu diesem Zeitpunkt ist aufgrund der Geometrie der Transfervorrichtung 1 der die Zentrierung herbeiführende Rücksprung 16 noch nicht in Kontakt mit dem Stopfen 5 bzw. der diesen umschließenden Kappe 6. Vielmehr liegt die Transfervorrichtung 1 mit einer konisch sich verjüngenden Einführschräge 22 des Randsabschnitts 13 entlang einer kurzen Linie bzw. einer Punktberührung an der Kappe 6 an. Beim weiteren Einführen des Glasfläschchens 2 in die Transfervorrichtung 1, wie es in Figur 4 veranschaulicht ist, wird über die Einführschräge 22 und den Rücksprung 6 das Glasfläschchen 2 mit dem Stopfen 5 bezüglich der Transfervorrichtung 1 zentriert. Da der axialen Einstechbewegung des Einstechdorns 10 eine radiale Bewegung überlagert ist, bildet sich beim Durchstechen des Stopfens 5 ein Riss 23 im elastischen Stopfen 5, der insbesondere als Gummistopfen ausgebildet ist. Wird das Glasfläschchen 2 weiter in die Transfervorrichtung 1 eingeschoben, bis der Rücksprung 16 die Kappe 6 im Bereich des Wulstes 4 des Glasfläschchens hintergreift, ist der Dichtabschnitt 21 des Einstechdorns 10 in einer Position bezüglich des Stopfens 5, dass er diesen nicht nur berührt, sondern stirnseitig in den Stopfen 5 eingedrungen ist. Die Stirnseite 24 des Dichtabschnittes 21 kontaktiert damit den Stopfen 5 ringförmig. Die Abstufung von Dichtabschnitt 21 und Einstechabschnitt 20 ist so gewählt, dass in der vollständig in die Transfervorrichtung 1 eingesetzten Stellung des Glasfläschchens 2, wie es in Figur 5 gezeigt ist, der Riss 23 vom Dichtabschnitt 21 abgedichtet wird.

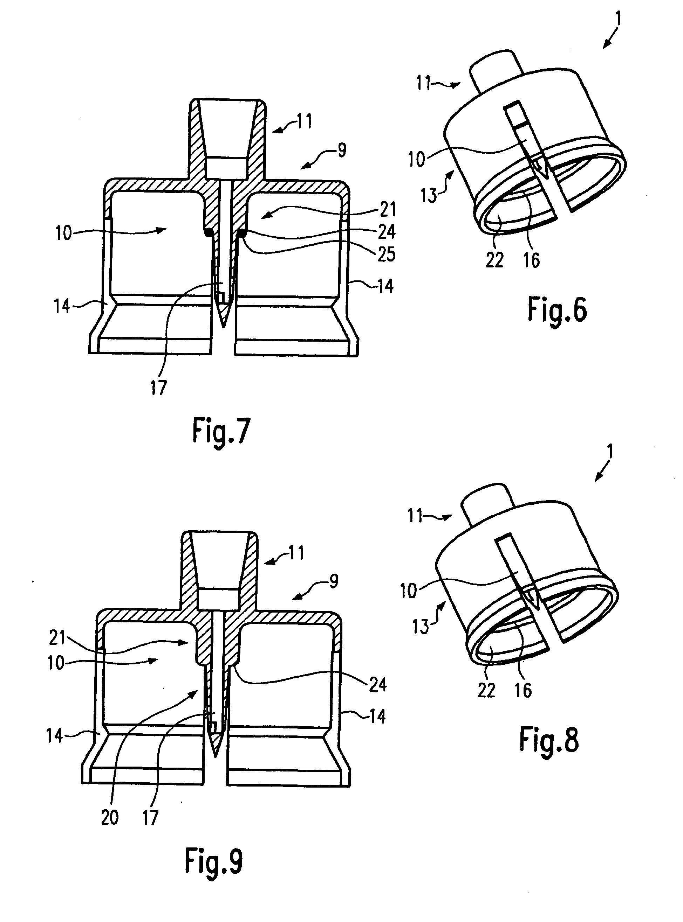

Das unbeanspruchte Beispiel nach den Figuren 6 und 7 unterscheidet sich von derjenigen, nach den Figuren 1 bis 5 dadurch, dass in die Stirnseite 24 des Dichtabschnittes 21 ein O-Ring 25 eingesetzt ist. Bei dieser Ausführungsform kontaktiert somit der elastische O-Ring 25 den elastischen Stopfen 5, im Unterschied zur Ausführungsform nach den Figuren 1 bis 5, bei der der elastische Stopfen 5 von dem nicht elastischen Dichtabschnitt 21 kontaktiert wird.

Das unbeanspruchte Beispiel nach den Figuren 8 und 9 unterscheidet sich von derjenigen, nach den Figuren 1 bis 5 dadurch, dass der Einstechabschnitt 20, von der Spitze des Einstechdorns 10 ausgehend, sich konisch erweitert. Beim Einstecken des Glasfläschchens 2 in die Transfervorrichtung 1 im Sinne der Funktionsdarstellung gemäß der Figuren 3 bis 5, ergibt sich somit bereits dann, wenn der Einstechdorn 10 in den Stopfen 5 eindringt, eine gewisse Dichtwirkung im Bereich des Risses 23 mittels des konisch gestalteten Einstechabschnittes 20. In der in die Transfervorrichtung 1 vollständig einsteckten Stellung des Glasfläschchens 2 erfolgt die zusätzliche Abdichtung über die Stirnseite 24 des Dichtabschnittes 21.

### Bezugszeichenliste

- 1: Transfervorrichtung
- 2: Glasfläschchen
- 3: Flaschenhals
- 4: Wulst
- 5: Stopfen
- 6: Kappe
- 7: Öffnung
- 8: Ausnehmung
- 9: Aufnahmekappe
- 10: Einstechdorn
- 11: Anschlussstutzen
- 12: Ausnehmung
- 13: Randabschnitt
- 14: Lappen
- 15: Deckelabschnitt
- 16: Rücksprung
- 17: Strömungskanal
- 18: Öffnung
- 19: Raum
- 20: Einstechabschnitt
- 21: Dichtabschnitt
- 22: Einführschräge
- 23: Riss
- 24: Stirnseite
- 25: O-Ring

## Patentansprüche

1. Transfervorrichtung (1), insbesondere für ein im Inneren unter Unterdruck stehendes Behältnis (2), mit einer Aufnahmekappe (9) zur Aufnahme eines mittels eines elastischen Stopfens (5) verschlossenen Wulstes (4) des Behältnisses (2), wobei die Aufnahmekappe (9) einen Randabschnitt (13) zum Zentrieren des Wulstes (4) in dessen in die Aufnahmekappe (9) eingesteckten Stellung und einen Deckelabschnitt (15) aufweist, mit dem Deckelabschnitt (15) ein zentraler Einstechdom (10) verbunden ist, der in den von der Aufnahmekappe (9) umschlossenen Raum (19) ragt, wobei der Einstechdorn (10) beim Einstecken des Wulstes (4) in die Aufnahmekappe (9) den Stopfen (5) durchsticht, wobei der Einstechdorn (10) einem diesen längs durchsetzenden Strömungskanal (17) für ein Fluid aufweist, der durch den Deckelabschnitt (15) nach außen geführt ist, und wobei der Einstechdom (10), gezogen auf dessen Einstechrichtung, einen vorderen Einstechabschnitt (20) und einen hinteren, im Durchmesser größeren Dichtabschnitt (21) aufweist, **dadurch gekennzeichnet, dass** die Länge des Dichtabschnittes (21) derart bemessen ist, dass der Dichtabschnitt (21), in der in die Aufnahmekappe (9) eingesteckten Stellung des Wulstes (4), in den Stopfen (5) eindringt und dass sich der Dichtabschnitt (21) als konische Erweiterung an den Einstechabschnitt (20) anschließt.

2. Vorrichtung nach Anspruch 1, **dadurch gekennzeichnet, dass** sie rotationssymmetrisch ausgebildet ist.

3. Vorrichtung nach einem der Ansprüche 1 bis 2, **dadurch gekennzeichnet, dass** der Randabschnitt (13) der Aufnahmekappe (9) einen Rücksprung (16) zum Hintergreifen des Wulstes (4) in der in die Aufnahmekappe (9) eingesteckten Stellung des Wulstes (4) aufweist.

4. Vorrichtung nach Anspruch 3, **dadurch gekennzeichnet, dass** der axiale Abstand (A) von Rücksprung (16) und Dichtabschnitt (21) kleiner ist als der axiale Abstand (B) von Rücksprung (16) und dem Deckelabschnitt (15) zugewandter Fläche des Stopfens (5) bei in die Aufnahmekappe (9) eingesteckter Stellung des Wulstes (4).

5. Vorrichtung nach einem der Ansprüche 1 bis 4, **dadurch gekennzeichnet, dass** der Einstechabschnitt (20) sich zum Dichtabschnitt (21) hin konisch erweitert.

## Claims

1. Transfer device (1), in particular for a container (2) whose interior is under negative pressure, with a receiving cap (9) for receiving a bead (4) of the container (2) closed by means of an elastic stopper (5), the receiving cap (9) having an edge portion (13) for centering the bead (4) in its position of insertion in the receiving cap (9), and a lid portion (15), and with a central piercing mandril (10) which is- connected to the lid portion (15) and projects into the space (19) enclosed by the receiving cap (9), the piercing mandril (10) piercing the stopper (5) when the bead (4) is inserted into the receiving cap (9), and the piercing mandril (10) having a flow channel (17) extending through it for a fluid which is conveyed outward through the lid portion (15), and the piercing mandril (10), relative to its direction of piercing, having a front piercing portion (20) and a rear sealing portion (21) which is of greater diameter, **characterized in that** the length of the sealing portion (21) is dimensioned such that the sealing portion (21) penetrates into the stopper (5) when the bead (4) is in its position of insertion in the receiving cap (9) and **in that** the sealing portion (21) is a conical widening which adjoins the piercing portion (20).

2. Device according to Claim 1, **characterized in that** said device is of a rotationally symmetrical design.

3. Device according to either of Claims 1 and 2, **characterized in that** the edge portion (13) of the receiving cap (9) has an inward projection (16) for engaging behind the bead (4) when the bead (4) is in its position of insertion in the receiving cap (9).

4. Device according to Claim 3, **characterized in that** the axial distance (A) between inward projection (16) and sealing portion (21) is smaller than the axial distance (B) between inward projection (16) and that surface of the stopper (5) facing the lid portion (15) when the bead (4) is in its position of insertion in the receiving cap (9).

5. Device according to one of Claims 1 to 4, **characterized in that** the piercing portion (20) widens conically toward the sealing portion (21).

## Revendications

1. Dispositif de transfert (1), en particulier pour un récipient (2) se trouvant intérieurement sous dépression, avec un capuchon de réception (9) destiné à recevoir un rebord (4) du récipient (2) fermé au moyen d'un bouchon élastique (5), dans lequel le capuchon de réception (9) présente une partie de bord (13) pour le centrage du rebord (4) dans sa position engagée dans le capuchon de réception (9) et une partie de couvercle (15), un mamelon plongeant central (10) est relié-à la partie de couvercle (15) et pénètre dans l'espace (19) entouré par le capuchon de réception (9), dans lequel le mamelon plongeant (10) transperce le bouchon (5) lors de l'engagement du rebord (4) dans le capuchon de réception (9), dans lequel le mamelon plongeant (10) présente un canal d'écoulement (17) pour un fluide qui le traverse en longueur, et qui est conduit vers l'extérieur à travers la partie de couvercle (15), et dans lequel le mamelon plongeant (10) présente, par rapport à sa direction d'engagement, une partie plongeante avant (20) et une partie d'étanchéité arrière (21) de plus grand diamètre, **caractérisé en ce que** la longueur de la partie d'étanchéité (21) est dimensionnée de telle manière que la partie d'étanchéité (21) pénètre dans le bouchon (5) lorsque le rebord (4) se trouve dans la position engagée dans le capuchon de réception (9), et **en ce que** la partie d'étanchéité (21) se raccorde à la partie plongeante (20) sous la forme d'un élargissement conique.

2. Dispositif selon la revendication 1, **caractérisé en ce qu'**il est de forme symétrique de révolution.

3. Dispositif selon l'une quelconque des revendications 1 à 2, **caractérisé en ce que** la partie de bord (13) du capuchon de réception (9) présente un retrait (16) pour l'accrochage du rebord (4) dans la position du rebord (4) engagée dans le capuchon de réception (9).

4. Dispositif selon la revendication 3, **caractérisé en ce que** la distance axiale (A) du retrait (16) à la partie d'étanchéité (21) est plus petite que la distance axiale (B) du retrait (16) à la face du bouchon (5) tournée vers la partie de couvercle (15) dans la position du rebord (4) engagée dans le capuchon de réception (9).

5. Dispositif selon l'une quelconque des revendications 1 à 4, **caractérisé en ce que** la partie plongeante (20) s'élargit en forme conique en direction de la partie d'étanchéité (21).
